# EUROPEAN PATENT SPECIFICATION

(11) **EP 0 487 660 B1**
(45) Date of publication and mention of the grant of the patent: **20.08.1997**
(21) Application number: 91907925.1
(22) Date of filing: 08.04.1991
(51) Int. Cl.: C12N 9/64, A61K 38/46

(54) **TREATMENT OF THROMBOTIC EVENTS**
BEHANDLUNG THROMBOTISCHER EREIGNISSE
TRAITEMENT DE LA THROMBOSE

(30) Priority: 06.04.1990 GB 9007879; 20.04.1990 GB 9008863
(43) Date of publication of application: 03.06.1992
(73) Proprietor: MERCK PATENT GmbH, D-64271 Darmstadt (DE)
(72) Inventor: SAWYER, Roy, T. Gorwelion Trapp, Dyfed SA19 6TR (GB); POWELL-JONES, Christopher 47 Heol Cennen, Llandeilo Dyfed SA19 6UL (GB); ATKINSON, Anthony Twingley Mill Corner, Salisbury Wiltshire SP4 6JJ (GB); ELECTRICWALA, Asgar 6 Ladysmith East Gomeldon, Wiltshire SP4 6LD (GB)
(86) International application number: GB9100549
(87) International publication number: WO9115576

(56) References cited:
- WO-A-89/09615
- US-A- 4 390 630
- PLATELETS, vol. 2, 01 March 1991, Longman Group UK Ltd.; R. MUNRO et al., pp. 55-56
- JOURNAL OF LABORATORY & CLINICAL MEDICINE, vol. 103, no. 1, January 1984; S.M. MALINCONICO et al., pp. 44-58
- JOURNAL OF CHROMATOGRAPHY, vol. 502, no. 2, 02 March 1990, Elsevier Science Publishers B.V., Amsterdam (NL); J.K. SWADESH et al., pp. 359-369
- JOURNAL OF LABORATORY & CLINICAL MEDICINE, vol. 104, no. 5, November 1984, C.V. Mosby Co., St. Louis, MO (US); S.M. MALINCONICO et al., pp. 842-854

## Description

This invention relates to a novel use of a polypeptide called hementin for manufacturing a medicament for the therapeutic dissoultion of pre-fonned platelet aggregates, including platelet-rich clots in whole blood and in vivo. The medicament comprising said polypeptide can be administered to patients suffering from thrombotic diseases such as heart disease and stroke.

Thrombosis is the disease process which culminates in the formation of a blood clot (thrombus or embolism) which blocks (occludes) a blood vessel supplying blood to a vital organ, such as the heart or brain. Unless these thrombi are treated, the result can be death by heart attack (myocardial infarction), pulmonary embolism or stroke. In addition to these life threatening forms of thrombosis, there is a plethora of minor thrombotic events and conditions which are mainly due to inappropriate formation of clots in the vasculature. Examples of such events are deep vein thrombosis and, more seriously, the thrombosis caused by bacterial infection resulting in septic shock. The mechanism by which thrombosis occurs is very complex and not completely understood but is believed in some cases to be initiated by the exposure of a diseased part of the blood vessel wall (atheromatous lesion) to the normal healing process of blood clotting (haemostasis) which involves the accumulation at the site of injury of blood components such as platelets and fibrin deposits.

At some point, unless treatment is given, such a thrombus grows sufficiently large either to occlude the vessel in which it is formed or it breaks off and travels in the blood stream until it blocks a blood vessel of smaller diameter (this phenomenon being known as embolism).

At present treatment for thrombotic disease is largely confined to two well-known forms of therapy, as follows: 1. prevention of clot formation with anti-coagulants, and 2. cleavage of a fibrin clot (by fibrinolysis or thrombolysis) by administration of a plasminogen activator such as tissue plasminogen activator (TPA) in order to activate the production of plasmin from plasminogen.

However, a material has not as yet been identified which can, in a therapeutically effective amount, specifically dissolve or deaggregate platelet aggregates, which often occlude blood vessels in the manner described above. (A therapeutically effective amount is an amount which would be acceptable to a patient, with acceptably low risk of adverse side effects, such as general proteolysis.)

The linkages between the platelets present in platelet aggregates generally comprise fibrinogen, but there is no direct correlation such that materials having fibrinogenolytic properties are necessarily capable of platelet aggregate deaggregation. Thrombolytic agents without the ability to deaggregate platelet aggregates have been identified and it is known that many fibrinogenolytic enzymes (although capable of dissolving fibrinogen) cannot deaggregate platelet aggregates. For example, plasminogen activators such as streptokinase or urokinase do not deaggregate platelet aggregates and neither do N-terminal fibrinogenases such as thrombin or fibrinogenases isolated from snake venom.

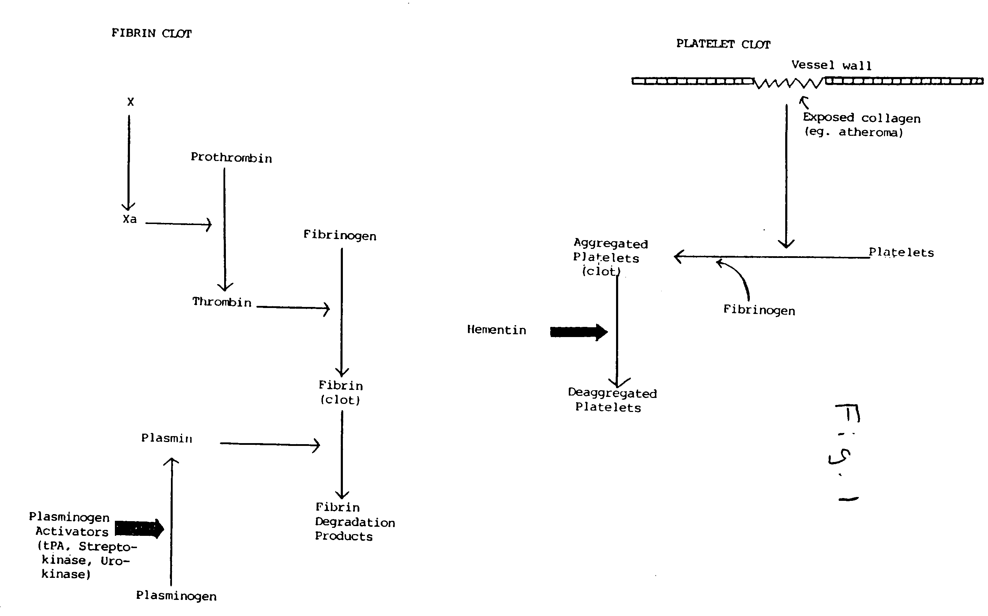

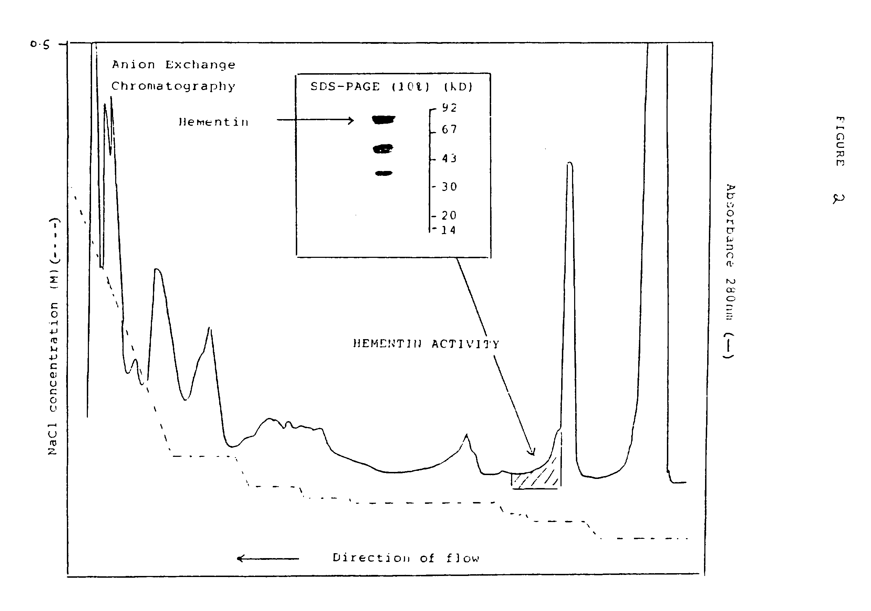

The differing roles, and hence modes of action, of hementin and plasminogen activators, in respectively breaking up fibrin clots and platelet aggregates, are illustrated in Figure 1. Plasminogen activators promote the conversion of plasminogen to plasmin which acts on fibrin clots; in contrast hementin has been found to act on the fibrinogen linkages of the platelet aggregates so as to cause subsequent deaggregation of the aggregates by preferential cleavage.

Deaggregation of platelet aggregates was thought to be impossible by treatment of the aggregates with a specific enzyme (such as a fibrinogenase) in a therapeutically effective manner. TPA is known in excessively high concentrations (i.e. in substantial excess of a therapeutically effective amount) to promote deaggregation of platelet aggregates by proteolysis by plasmin of fibrinogen-crosslinked platelets. However, TPA does not selectively deaggregate platelet aggregates; furthermore it has an adverse effect on other clotting factors in the circulation system (such as Factor X) and can cause lysis of haemostatic plugs which arise in repair areas in the vasculature. It has been suggested that this non-specific action of plasminogen activators may be the cause of haemorrhaging which often occurs with such thrombolytic agents. The action of TPA is analogous to that of an unspecific protease and is not specific to the fibrinogen linkages of the platelet aggregates.

Another factor precluding the use of TPA to deaggregate platelet aggregates in vivo is that it would be necessary to administer an unacceptably high level to a patient for the administered TPA to have the desired therapeutic effect. Furthermore, TPA is inhibited by plasma and it would therefore be necessary to administer the TPA as a continuous perfusion for it to retain its deaggregating activity against platelet aggregates.

Various materials having activity in mammalian cardiovascular systems are known to be present in leech secretions; amongst these is hementin, a fibrinolytic enzyme derived from leeches of the order Rhynchobdellida, such as the leech species Haementeria ghilianii. Hementin, and the isolation thereof, is described in U.S. Patent 4390630.

The fibrinogenolytic activity of hementin is described by Malinconico S.H.; Katz J.B. and Budzynski A.Z. in "Fibrinogen degradation by hementin - a fibrinogenolytic anticoagulant from the salivary glands of the leech Haementeria ghilianii", in J. Lab. Clin. Med. 1984; 104(5) pp842-854. There is no hint or suggestion in either this article, or in the abovementioned U.S. patent 4390630, of hementin having the unique ability relative to other fibrinogenolytic enzymes, to deaggregate platelet aggregates.

This invention discloses now an unexpected property for compositions comprising hementin, which is the ability in vivo of such compositions in therapeutically effective amounts, to specifically dissolve platelet aggregates (including platelet-rich clots) via fibrinogen cleavage, to the exclusion of other coagulation factors and blood clots.

Therefore, it is an object of this invention to make available a novel use of a polypeptide called hementin for manufacturing a medicament (comprising that polypeptide) for the therapeutic dissoultion of pre-formed platelet aggregates, including platelet-rich clots in whole blood and in vivo, the polypeptide for this is obtainable by homgenizing tissue from the salvary glands of the leech *Heamenteria ghilianii* with solution buffers having salt concentrations of 15 to 25 mM at pH value between 7 and 8, and purification of the homogenate by ion-exchange chromatography with an eluent having a salt concentration of 80 to 150 mM, and optionally by a gel filtration step.

The precise mechanism by which hementin deaggregates platelet aggregates and hence dissolves platelet rich clots appears to be unique. All other fibrinogenolytic enzymes cleave preferentially either:
1. at the COOH-terminal of the exposed Aa chain of fibrinogen, e.g. plasmin and some snake venoms (this exposed Aa chain is also susceptible to enzyme attack by general proteases such as trypsin), or
2. at the NH₂-terminal of the fibrinogen molecule e.g. thrombin and some snake venoms, which are primarily concerned with removal of fibrinopeptide A and/or fibrinopeptide B (or similar short peptide fragments), the removal of which usually results in the formation of a component which is still capable of polymerising and hence forming a clot predominantly comprising fibrin.

Hementin is the only known enzyme which cleaves first through all the three chains (Alpha, Beta and Gamma) in the connector region between the D and E domains (Kirschbaum, N.E. and Budzynski, A.Z. 1990. "A unique proteolytic fragment of human fibrinogen containing the Aa COOH-terminal domain of the native molecule"; J..Biol. Chem 265: 13669-13676) and which (in a therapeutically effective amount) can subsequently deaggregate platelet aggregates. It has been postulated that cleaving at this site is the most accessible and most effective for breaking the bivalent nature of fibrinogen bound to platelets (Sawyer, R.T., Powell-Jones, C. and Munro, R. 1991. The biological function of hementin in the proboscis of the Amazon leech Haementeria ghilianii; Blood coagulation and Fibrinolysis Vol.2, pp153-159). There is no hint or suggestion of the primary structure of hementin in the above mentioned patents or papers.

The platelet aggregates treated with the polypeptide (hementin) according to the invention may be blood clots which might otherwise block blood vessels thereby causing illness by heart attack, pulmonary embolism or stroke or other major or minor manifestation of impaired blood supply.

Patients suffering from pulmonary embolism typically experience accelerated Erythrocyte Sedimentation Rate (ESR). Administration of a composition comprising hementin according the present invention, to such patients, has now been shown to reduce accelerated ESR. Furthermore, the action of hementin-containing compositions to reduce ESR also demonstrates that accelerated ESR is in some way related to erythrocyte interaction with fibrinogen. Hementin can therefore serve as a diagnostic index to the degree of erythrocyte interaction with fibrinogen in individual patients.
Additionally, it has also been found that, where increased plasma fibrinogen affects blood viscosity, hementin compositions can be used therapeutically to reduce the viscosity.

A further advantageous property of hementin is that its in vivo activity in deaggregating platelet aggregates is significantly enhanced relative to its in vitro activity. This is completely opposite to the relative general activities of TPA in vivo and in vitro; TPA has a much lower general activity in vivo compared to in vitro due to inhibition in vivo by plasma.

The composition used in treating such thrombotic events may be derived directly from leech tissue or leech secretions of leeches of the order Rhynchobdellida (for example, from salivary glands of Haementeria ghilianii), or optionally, it may be further purified (for example, to essentially pure hementin, which is characterised by having a single primary protein peak and specific activity greater than 1000 u/mg protein).
Alternatively, it may be derived indirectly by genetic manipulation and replication techniques from such tissue or secretions (and, as explained hereinafter, it is only now that we have elucuidated the amino acid sequence of hementin that such manipulation and replication techniques has become possible). Typically, cloned material is indirectly derived from leech tissue or leech secretions of leeches of the order Rhynchobdellida and expressed from a bacterial host in which the hementin is replicated.

Previously, only a short amino acid sequence of hementin was known, which was insufficient to allow the construction and replication of a unique oligonucleotide probe. The reported sequence for the first 10 amino acids at the N-terminal end of hementin from the anterior salivary glands was reported to be as follows (Swadesh, J.K., Huang, T.Y. and Budzynski, A.Z. 1990. "Purification and characterisation of hementin, a fibrinogenolytic protease from the leech Haementeria ghilianii"; J. Chromatography 502: 359-369):

1                   5                        10
thr-thr-leu-thr-glu-pro-glu-pro-asp-leu-

This invention provides now the amino acid sequence of hementin which makes possible replication thereof.

Thereofore, it is further object of the invention to provide the N-terminal sequence of hementin, isolated from either the anterior or posterior gland which is : 
1                    5                    10
thr-thr-leu-thr-glu-pro-glu-pro-pro(or phe or asn)-leu-thr-thr(o

                    15                    20
phe)-leu-thr(or asn)-phe-val-arg(or asp)-ile(or asn)-val-asn(or

                    25
lys)-val(or leu)-glu(or asp)-met-pro-ile-phe-val(or asp or gly o[?]

                    30                    35
ala or phe)-met-ala-thr(or arg)-ala-asn(or gln)-ser-gln-ile-

                    39
thr(or tyr)-ser(or thr or tyr)-thr(or lys)-phe-

(for the anterior); or

1                    5                    10                    15
thr-thr-leu-thr-glu-pro-glu-pro-phe-leu-thr-tyr-leu-thr-phe-val-

                    20
arg(or lys)-ile-val-asn(or lys)-val(or leu)-glu-met-pro-ile-phe-

          30                    35                    39
val-met-ala-thr-ala-asn-ser-gln-ile-thr-ser-thr-phe-
 (for the posterior).

An internal amino acid sequence of anterior gland hementin is 
 1               5                   10                 15
     *   *   *   *   *       *   *
gly-tyr-thr-asn-tyr-ala-lys-phe-leu-asp-tyr-leu-pro-val-glu-arg-

             20
gly-ile-pro-leu ,
 Portions of this sequence in positions 2-6 and 8-9 (indicated by asterisks), respectively, correspond to the sequence reported by Swadesh et al (1990. J. Chromatography 502: pp. 359-369) and designated a contaminating peptide in hementin purification. The sequence was: glu-val-try-thr-asn-tyr-ala-ser-phe-leu-Our sequence probably does not correspond to a contaminating peptide; the sequence may result from acid hydrolysis of asn-gly bond within the hementin molecule.

DNA base sequences coding for any of the above amino acid sequences can be extrapolated or determined by standard techniques; the present invention therefore comprises a DNA base sequence coding for any of the above new amino acid sequences.

The composition comprising hementin may be derived by homogenising dissected salivary glands (typically from leeches of the order Rhynchobdellida, such as Haementeria ghilianii or Haementeria officinalis, or other related species) in a suitable aqueous solvent, and purifying the active ingredients using a sequence of chromatographic operations.

There is therefore provided by the present invention a method of isolating a material capable of dissolving preformed platelet aggregates, which method comprises:-
(a) in the case where the starting material is leech tissue, homogenising at least the salivary glands of leeches of the order Rhynchobdellida in a Hepes buffer;
(b) purification of the homogenate of step (a), or secretions from the salivary glands of leeches of the order Rhynchobdellida in a Hepes buffer, or cloned hementin expressed from a bacterial source, by ion-exchange chromatography; and
(c) addition of a blocking agent to the eluate of step (b).

As indicated therefore, a similar method would be used to isolate the cloned, chemically identical hementin, from micro-organisms in which the hementin has been expressed, as would be used in isolation of hementin from leech tissue or secretions.

Preferably, sodium chloride buffer is used as eluant in step (b). The blocking agent in step (c) is to prevent purified hementin losing activity on lyophilization, freezing or thawing; an example of such a blocking agent is bovine serum albumin.

Typically, the method further comprises the use of a heparin Sepharose column to eliminate contamination of the product by inhibitor Factor Xa and also gel filtration chromatography to improve the purity of the product.

There is also provided by the present invention therefore a product obtainable by the isolation method as hereinbefore described, said product being capable, when administered in a therapeutically effective amount to an animal patient (such as a human patient), of selectively dissolving or deaggregating platelet aggregates. Preferably, the product comprises hementin, which is typically essentially pure hementin.

A small scale hementin isolation and purification procedure has previously been described (Swadesh, J.K., Huang, I.Y and Budzynski, A.Z. 1990. Purification and characterisation of hementin, a fibrinogenolytic protease from the leech Haementeria ghilianii; J. Chromatography 502: 359-369). However, the latter procedure uses a strong anion exchange process and an ammonium bicarbonate buffer and cannot be used for large scale or pharmaceutical use for the following reasons.
(1) The ammonium bicarbonate buffer in the presence of calcium chloride results in an insoluble precipitate, presumably calcium carbonates, upon lyophilization.
(2) The purified hementin loses most of its activity upon freezing and thawing, as well as lyophilization.
(3) The procedure can result in a product contaminated with detectable amounts of inhibitor of coagulation Factor Xa, known to occur in the salivary glands of the leech Haementeria ghilianii (Blankenship et al. 1990; Biochem. Biophys. Res. Comm. 166: 1384-1389).

The composition comprising hementin used in the method according to the invention in addition has anticoagulant and fibrinolytic activity, which (together with the activity on platelet aggregates) act additively or synergistically on one or more steps of the process of blood coagulation. Compositions comprising hementin, alone or in combination with other anti-coagulants, thrombolytic agents or anti-platelet agents, may also be used according to the invention for the prevention or therapeutic treatment of thrombosis.

A pre-requisite for platelet aggregation is the binding of fibrinogen to GpIIb-IIIa receptors on activated platelets. Activation by physiological agonists such as collagen, thrombin and ADP results in the exposure of binding sites for fibrinogen on the platelet surface resulting in the crosslinking of platelets through the Aa chains of fibrinogen bound to GpIIb-IIIa receptors on the activated platelets.

It has been found according to this invention that compositions comprising hementin have an effect on this process, and can not only prevent platelet aggregation but also result in platelet deaggregation.

It is possible to elicit extensive deaggregation of ADP induced platelet aggregates by means of compositions comprising hementin. Extensive deaggregation occurred using ADP but less effectively when high concentrations of other inducers such as collagen were tried. This may be because when these other inducers are used, types of platelet linkage may occur other than those utilising fibrinogen.

The ability of compositions comprising hementin to selectively deaggregate platelets is a property not shared by plasminogen activators such as TPA or streptokinase. This gives compositions comprising hementin a possible advantage in that they may have selectivity for platelet-rich thrombi which are refractory to such plasminogen activators. Therefore, because thrombi are complex multi-component structures of varying ages and composition, and anti-platelet therapy is likely to become a significant therapeutic strategy, the identification of the platelet directed mode of action of hementin is important progress towards providing new tools for the treatment of thrombotic disease.

According to a further aspect of the invention, therefore, there is provided a composition comprising hementin, for use in the manufacture of a medicament for the therapeutic deaggregation of platelet aggregates and optionally also for the inhibition or prevention of platelet aggregation.

There is also provided a composition comprising a material capable of selectively cleaving between D and E domains of the fibrinogen linkages present in said platelet aggregates, for use in the manufacture of a medicament for the therapeutic deaggregation of platelet aggregates and optionally also for the inhibition or prevention of platelet aggregation.

The present invention further comprises the use of compositions comprising hementin for the preparation of medicaments for administration to a patient together with a plasminogen activator, such as TPA, for clot lysis. Such administration may be without any further inhibitor of platelet aggregation.

The present invention still further comprises the use of compositions comprising hementin together with hirudin or other leech derived anti-coagulant (such as that disclosed in our prior PCT Patent Specification WO90/05143).

The present invention therefore further comprises a pharmaceutical formulation for the treatment of platelet aggregates, which comprises hementin, an inert carrier or excipient therefor, and, optionally, one or more further active materials, such as a leech-derived anti-coagulant. Such an anti-coagulant is preferably an anti-thrombin, which is typically a hirudin or hirudin analogue, or Factor Xa.

The carrier or excipient is preferably such that the formulation can be administered intravenously; an example of a suitable carrier is sterile saline. The intravenous formulation typically contains sufficient hementin that, when administered to a patient, the latter has 20 to 100 units of hementin per ml of blood.

It has been established by this invention that compositions comprising hementin have four anti-coagulant and anti-thrombotic properties which are effective by themselves or in combination with other drugs directed at similar targets. These properties may be summarised as:
1. Anti-coagulant - inhibition of the ability of blood to clot (in the case of purified hementin, this is believed to result from fibrinogenolytic activity which depletes fibrinogen available for clotting).
2. Fibrinolytic - an ability to dissolve fibrin clots.
3. Inhibitor of platelet aggregation - an ability to prevent aggregation initiated by a number of agonists via action on fibrinogen.
4. Platelet deaggregation - a unique and surprising ability to selectively deaggregate pre-formed platelet aggregates.

The present invention will now be described in more detail, with reference to the following examples, in which references to crude hementin relate to compositions having an activity of approximately 100 (typically 25-100) u/mg protein, while references to purified hementin relate to compositions having an activity greater than 1000 u/mg protein.

The unit activity of hementin is defined as the number of micrograms per millilitre of fibrinogen rendered incoagulable by thrombin per minute at 37 degrees Celsius.

The present invention also provides new more sensitive assays for detecting and measuring this hementin activity. Hementin is very salt dependent and consequently activity must be measured at near physiological levels of NaCl in the reaction buffer. The reaction conditions are: 50mM Hepes, 10mM CaCl₂, 0.15 mM NaCl, 0.1% Brij 35, pH 7.5. In contrast, in the paper mentioned above by Swadesh et al the assay buffer was 150mM Hepes, pH 7.9 and 10mM CaCl₂. The invention provides a sensitive assay based on analysis of fibrinogen breakdown products detected by HPLC.

There is further shown by the examples isolation and purification techniques for hementin and subsequent characterisation of its chemical and physical properties.

### Example 1

### Purification of hementin from the anterior salivary gland

The anterior glands of 117 hungry third fed Haementeria ghilianii were manually dissected and frozen on dry ice. The glands were crushed to a fine powder in dry ice and then homogenised and thawed in 20mM Hepes, 10mM CaCl₂, pH 7.5. The homogenate was centrifuged (1000 x g) to remove insoluble cell debris and then further centrifuged (10000 x g) and filtered (0.45 micron Filter). This material was the starting point for purification and was designated Stage I material.

Hementin was then purified by high performance anion-exchange chromatography. Stage I material was brought up to 10ml in Hepes buffer described above and 1-2ml added at a time to a DEAE-5PW column (1 x 5cm; Waters-Millipore). The activity eluted between 80-150mM NaCl using a linear salt gradient.

To the pooled active fractions was added 1 mg/ml bovine serum albumin ('Cohn Fraction 5', Sigma) before lyophilization or freezing. The results of a typical purification are as follows:

| | Specific Activity (Units/mg) | Total (Units) | Recovery % |
|---|---|---|---|
| Stage I | 292 | 16,600 | - |
| Stage II | 600 | 13,200 | 83 |

### Example 2

### Gel filtration purification of anterior salivary gland material

Post anion-exchange material (Stage II) was pooled as described in Example 1. The pool was applied to a molecular sieving column (Superdex 200; Pharmacia) using a buffer containing 20mM Hepes, 10mM CaCl₂, pH 7.5. The fractions containing activity were pooled. To the pool was added 1 mg/ml bovine serum albumin (BSA) prior to freezing or lyophilization. The pool was then freeze-dried and designated Stage III material. The results of a typical purification are as follows:

| | Specific Activity (Units/mg) | Total (Units) | Recovery % |
|---|---|---|---|
| Stage III | 1400 | 5,600 | 35 |

### Example 3

### Purification of hementin from the posterior salivary gland

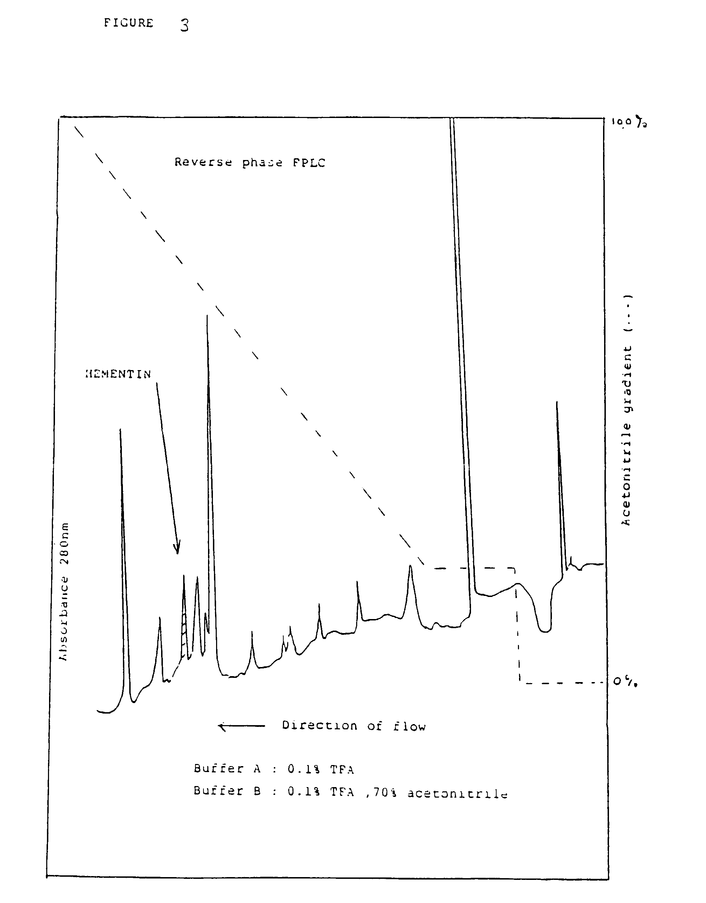

The posterior glands of 118 hungry fed Haementeria ghilianii were manually dissected and frozen on dry ice. Stage I and Stage II (post ion-exchange) material were prepared as in Example 1. During elution of the Stage II material from the anion-exchange column, aliquots of fractions containing activity were analysed by SDS-PAGE (10%) gels under reducing conditions (Figure 2 insert). To the active pool was added 1 mg/ml BSA prior to freezing or lyophilization.

The results of a typical purification were as follows:

| | Specific Activity (Units/mg) | Total (Units) | Recovery % |
|---|---|---|---|
| Stage I | 1,827 | 40,929 | |
| Stage II | 11,506 | 28,650 | 70 |

### Example 4

### Purification of anterior salivary hementin by HPLC

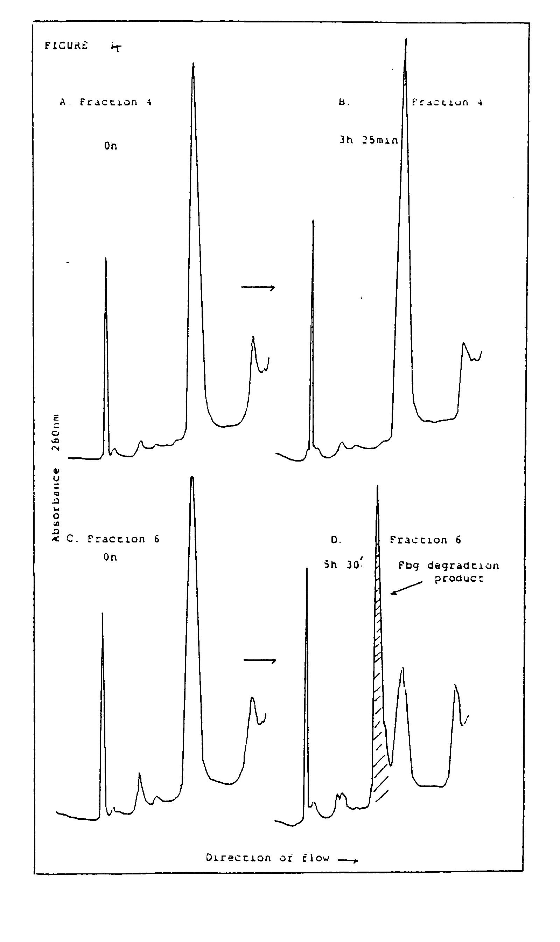

The anterior glands of 55 starved third fed Haementeria ghilianii were manually dissected and frozen on dry ice. The glands were homogenised in 10ml of 20mM Hepes, 10mM CaCl₂, pH 7.5 using acid-washed sand. The homogenate was centrifuged (10,000 x g) and the supernatant passed through a 0.43 micron filter. This was loaded onto an anion exchange column (DEAE-5P, Waters) and eluted using a salt gradient. Fractions were collected and assayed for activity and for protein (280nM). Activity was found in fractions 21-25 and in 29/30. Fractions 21 and 23-25 were pooled and used for further purification by reversed phase hydrophobic interaction chromatography (RP-HPLC). SDS-PAGE gels were run on fractions containing activity. The pooled active fractions from the anion exchange run were applied to a RP-HPLC column (C18, Waters) in 0.1% trifluoroacetic acid (TFA) and eluted with a gradient of 70% aqueous acetonitrile, 0.1% TFA (Figure 3). Fractions were collected and found to contain activity in fractions 53-55 which were freeze dried. The protein content was approximately 40ug SDS-PAGE analysis of the product under reducing conditions gave a molecular weight of about 80,000 which corresponds to that of hementin. Activity against fibrinogen was detected using SDS-PAGE analysis of fibrinogen fragments following incubation with hementin-containing fractions.

### Example 5

### Isoelectric Focussing of hementin

Knowledge of the precise isoelectric point (pI) value of proteins is useful in maximising the conditions for purification. In order to determine this characteristic property for hementin, the following experiment was conducted. The active fraction following application onto anion-exchange chromatography, as indicated in Figure 2, was further purified by FPLC on a Superose 12 gel filtration column, pre-equilibrated in 20mM Hepes buffer, pH 7.5 containing 0.2M NaCl, 10mM CaCl₂ and 0.1% Tween 80. 200ul of the sample was loaded onto the column and eluted with the above buffer at 0.4ml/min. The effluent was collected in fractions and its absorbance was monitored at 280nm. Two major protein peaks eluted at about 30 min and 40 min, respectively. Based on a fibrinogenolysis assay (as described in Example 12, below), the hementin peak was determined to be the one eluting at about 30 min.

This peak was concentrated and desalted on Sephadex G-25. After further concentration, an aliquot of the sample was analysed for its isoelectric point (pI) on a PhastGel 3-9 using a Pharmacia Phast System. The sample was applied centrally onto the gel. After staining with Coomassie Blue, one major band at approximately pI of 6.25 ± 0.1 was thought to correspond to hementin. Trace amounts of impurities could also be visualised on the gel at pI values below 4.6. Based on intensity of the stain, it is assumed that these impurities account for less than 5%. The pI of hementin is therefore approximately 6.25 ± 0.1.

### Example 6

### Fibrinogenolysis assay

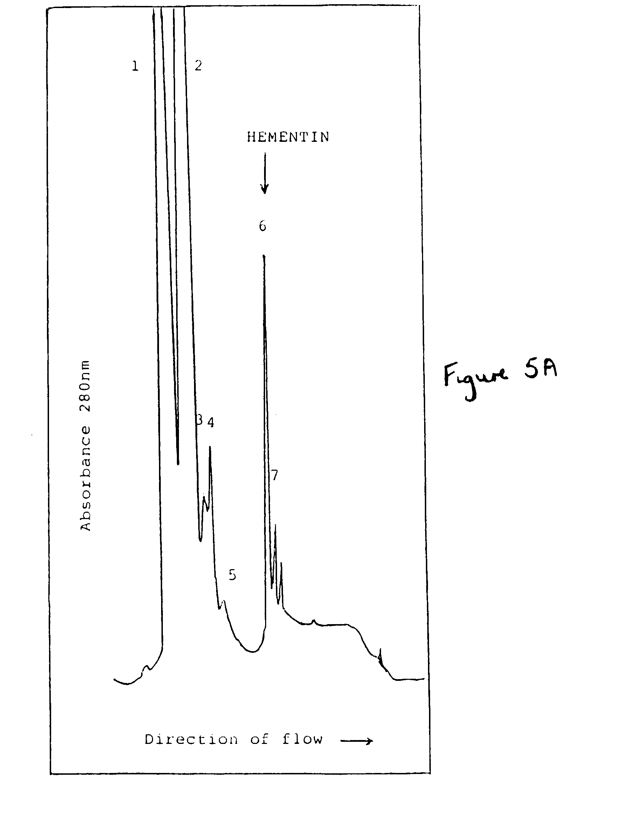

In order to recognise peaks of activity involving very limited amounts of material it was necessary to develop the following new, fast, convenient and very sensitive assay for fibrinogenolysis. The assay involves incubation of enzyme or purified column fractions with fibrinogen followed by rapid analysis of fibrinogen breakdown products on HPLC (RP300-C8). Stage II enzyme (50 microlitres) or column fractions were incubated in a Reacti-Vial with 50 microlitres of 50mM HEPES buffer, pH 7.5, 2mM CaCl₂, 50 microlitres of 0.15mM NaCl and 50 microlitres of fibrinogen (5mg/ml). Of this mixture (20 microlitres) was diluted with 30 microlitres 20 mM HEPES buffer and the total volume was immediately injected onto an ABI 130A analytical chromatograph with an RP30-CB column to produce a zero time chromatogram. The reaction mixture was eluted with a gradient between 0.1% TFA in water (solvent A) and 0.1% TFA in 80% aqueous acetonitrile (solvent B). The column was equilibrated at 35% B followed by a 5 minute rise to 45% B and a 20 minute gradient 50-55% B. The rest of the vial was incubated at 37 degrees Celsius for a desired period, such as half an hour, which was sufficient to detect activity in the form of changed HPLC elution profile due to the presence of fibrinogen degradation product (Figure 4). Fibrinogen alone eluted as a single peak from the HPLC column at 52% of solvent B. After 30 minutes incubation at 37 degrees Celsius with hementin the fibrinogen peak had been converted (90%) to an earlier elution peak peak (50%B) and one that appeared to elute later (53%B) than the original fibrinogen.

Inactive materials did not change the elution pattern for as much as 24 hours.

### Example 7

### Purification of anterior gland hementin by HPLC

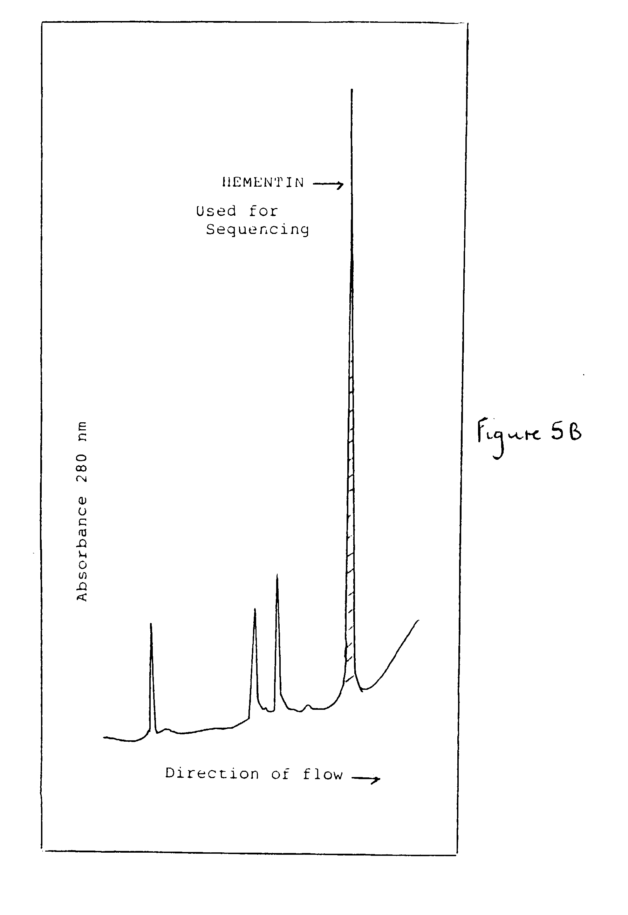

Stage II material, such as that described in Example 1, was purified by HPLC on a RP300 (C8) column. Solvent A was 0.1% TFA in water, Solvent B was 0.1% TFA, 80% aqueous acetonitrile. The gradient was 25 to 65% Solvent B in 30 min. Upon elution the material separated into 7 fractions as shown in the elution profile (Figure 5A). Under the assay conditions fractions 6 and 7 showed activity, as determined by the sensitive assay described in Example 6, whereas all other fractions were inactive. Fraction 6 contained the major activity.

### Example 8

### Amino acid composition of anterior gland hementin

Amino acid analyses were performed after 24 hour vapour phase HCl hydrolysis and modification with phenyl isosolcyocyanate (PITC).

Pooled Peak 6 (as shown by peak 1 in Figure 5B), prepared as described in Example 7, was put onto a Waters 840 amino acid analysis system (Picotag). Multiple regression analysis gave a minimum molecular weight of 83,000 as the best fit factor for all amino acid values, calculated by a computer programme of Black and Hogness. The amino acid composition of anterior gland hementin was determined to be as follows:

asp )
    ) | 87.89
asn )
thr | 31.89
ser | 78.94
glu )
    ) | 80.79
gln )
pro | 7.99
gly | 100.32
ala | 42.02
1/2cys | 25.08
val | 31.07
met | 27.23
ile | 30.04
leu | 59.70
tyr | 29.15
phe | 28.85
his | 26.93
lys | 42.24
trp | -
arg | 34.40

### Example 9

### N-terminal Amino Acid Sequence of Anterior Gland Hementin

In preparation for amino acid sequence analysis, Peak 6 hementin, prepared as per Example 7, was collected manually in dichlorodimethylsilane-treated glass tubes. The gradient was run at 100 microlitres/min and Peak 6 was collected in less than 200 microlitres in one tube. The pooled Peak 6 hementin was immediately concentrated to less than 100 microlitres and then transferred in 20 microlitres portions to a polybrene-treated membrane of an Applied Biosystems 477A pulse-liquid phase protein sequencer. For sequence analysis the fractions were collected without Hepes buffer. The N-terminal sequence, SEQ ID No:1, was determined as follows:

1                   5                                       10
thr-thr-leu-thr-glu-pro-glu-pro-pro(or phe or asn)-leu-thr-thr(or

                    15                                          20
phe)-leu-thr(or asn)-phe-val-arg(or asp)-ile(or asn)-val-asn(or

                                        25
lys)-val(or leu)-glu(or asp)-met-pro-ile-phe-val(or asp or gly

                              30                                35
or ala or phe)-met-ala-thr(or arg)-ala-asn(or gln)-ser-gln-ile-

                                                39
thr(or tyr)-ser(or thr or tyr)-thr(or lys)-phe-

### Example 10

### Internal Amino Acid Sequence of Anterior Gland Hementin

Pooled Peak 6 hementin, prepared as per Example 7, upon trifluoroacetic acid hydrolysis yielded a peptide fragment, SEQ ID No:3, with the following sequence:

 1               5                   10                  15
     *   *   *   *   *       *   *
gly-tyr-thr-asn-tyr-ala-lys-phe-leu-asp-tyr-leu-pro-val-glu-arg-

    20
gly-ile-pro-leu

Portions of this sequence in positions 2-6 and 8-9 (indicated by asterisks), respectively, correspond to the sequence reported by Swadesh et al (1990. J. Chromatography 502: pp.359-369) and designated a contaminating peptide in hementin purification. Their sequence was:

glu-val-tyr-thr-asn-tyr-ala-ser-phe-leu-

Our sequence probably does not correspond to a contaminating peptide, but to result from acid hydrolysis of an asn-gly bond within the hementin molecule.

### Example 11

### Amino Acid Sequence of Posterior Gland Hementin

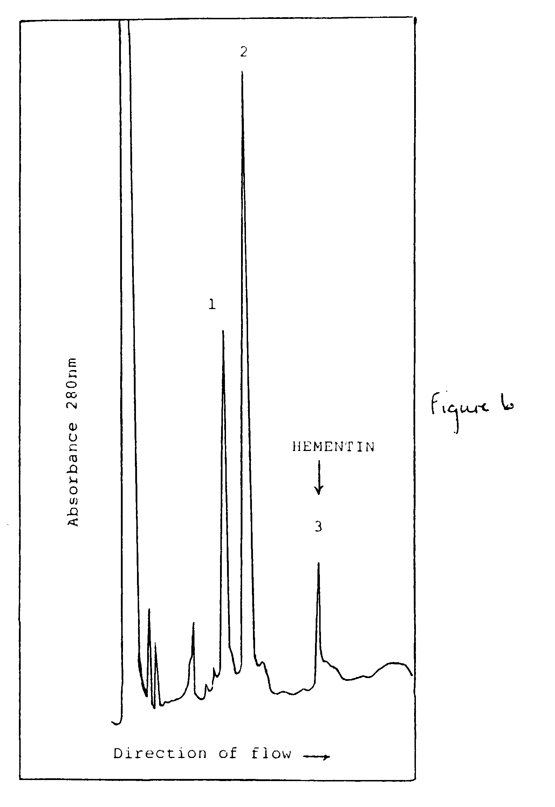

Stage II hementin prepared from the posterior glands as described in Example 3 were purified by HPLC as described in Example 7. The protein profile was different from that prepared from anterior gland Stage II hementin, with three major peaks (Figure 6). Activity was found in Peak 3. Pool fractions corresponding to the active peak (Peak 3) were put onto a pulse-liquid phase protein sequencer Applied Biosystems 477A. The N-terminal sequence SEQ ID No:2, was determined to be as follows:

1                    5                         10                        15
thr-thr-leu-thr-glu-pro-glu-pro-phe-leu-thr-tyr-leu-thr-phe-val-

                              20
arg(or lys)-ile-val-asn(or lys)-val(or leu)-glu-met-pro-ile-phe-

                  30                        35                    39
val-met-ala-thr-ala-asn-ser-gln-ile-thr-ser-thr-phe-

### Example 12

### Anti-coagulant effect.

The plasma anti-coagulation rate by crude hementin at a dose of 30u/ml (units/ml) was followed by measuring the prothrombin time (PT) using the Manchester reagent (Thrombosis Research Foundation, Stockport, UK); activated partial thromboplastin time (APTT) with kaolin/Bell and Alton platelet substitute (Diagnostic Reagents, Thame, UK); thrombin clotting time (TCT) with bovine thrombin (Baxter Healthcare, Newbury, Berks) and atroxin clotting time - a purified extract of Bothrops atrox venom (Sigma) used at a concentration of 0.5mg/ml. The PT, TCT, and APTT were measured in citrated plasma.

The results of these studies with crude hementin were as follows:

| Test | Time to incoagulate PRP (min) | control clotting time |
|---|---|---|
| PT | < 1.0 | 15 sec. |
| APTT | < 1.0 | 40 sec. |
| TCT | < 2.5 | 15 sec. |
| Atroxin | 15-20 | 17 sec. |

This shows the rapid effect of crude hementin on the coagulability of plasma.

The results with purified hementin on the coagulation of citrated plasma at 70u/ml were as follows:

| | PT | atroxin | TCT | APTT |
|---|---|---|---|---|
| CONTROL | 19 | 25 | 15 | 55 |
| 5min | 18 | 26 | 15 | 54 |
| 10min | 20 | 26 | 15 | 53 |
| 20min | >300 | 60 | >300 | >300 |
| 30min | - | >300 | - | - |

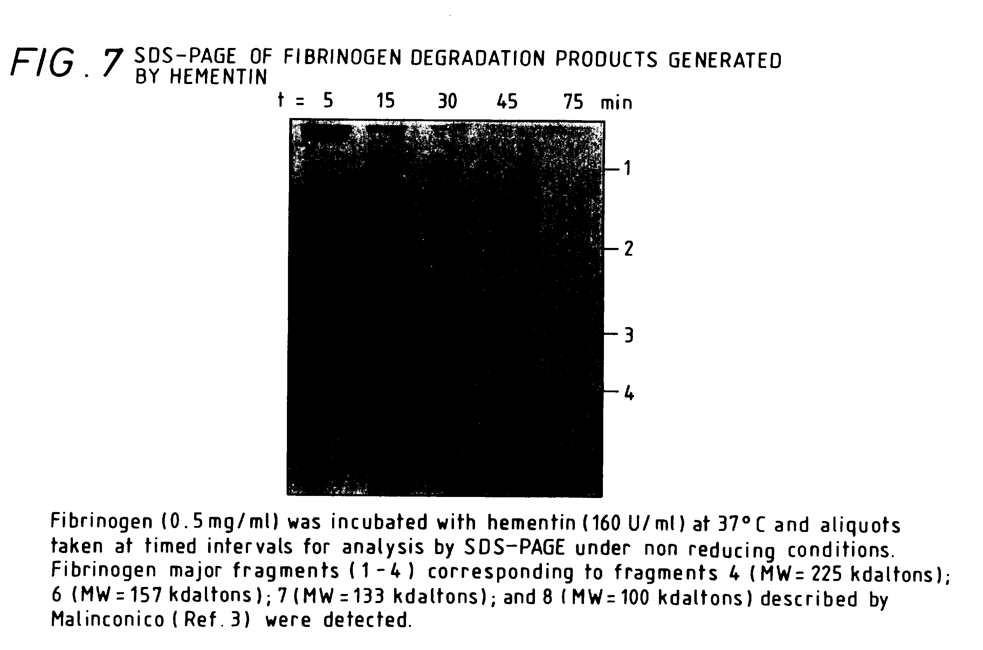

The effect of purified hementin on fibrinogen is shown in Fig.7, which shows the results of SDS-polyacrylamide gel electrophoresis, giving the times of generation of fibrinogen fragments and their molecular size. Fibrinogen (0.5mg/ml) was incubated with purified hementin (160 u/ml) at 37 degrees Celsius and aliquots taken at timed intervals for analysis by SDS-PAGE under non-reducing conditions. Fibrinogen major fragments (1-4) corresponding to Fragments 4 (MW 225 kdaltons); 6 (MW 157 kdaltons); 7 (MW = 133 kdaltons); and 8 (MW = 100 kdaltons) described by Malinconico were detected.

### Example 13

### Measurement of Platelet Aggregation

Crude hementin was extracted from the anterior glands of Haementeria ghilianii. The lyophilized crude hementin was reconstituted with isotonic saline prior to use for platelet studies or in 50mM Hepes, 10mM CaCl₂, 0.1% Brij 35, pH 7.5 for in vitro assay.

Freshly obtained blood samples were collected by venepuncture from the antecubital vein of 5 healthy human volunteers who had received no medication nor alcohol for at least two weeks. Paired samples from each volunteer were immediately placed in siliconised vessels containing two different anticoagulants - 1:9 v/v 0.105M sodium citrate or heparin (at a concentration which did not result in spontaneous platelet clumping - 20 IU/ml blood). Samples were centrifuged at 200g for 10min and harvests of platelet rich plasma (PRP) 9 were prepared to contain 250-350 x 10 per litre of platelets.

Where necessary, PRP was diluted with autologous platelet poor plasma (PPP). All test procedures were performed within the second hour following venesection. Platelet aggregation and deaggregation studies were performed in a four channel aggregometer at 37 degrees Celsius. Acid soluble Type I collagen (Chronolog Corp., Havertown, PA), ADP (Sigma Chemical Co, Poole, Dorset) and, in the case of citrated samples - bovine thrombin (Baxter Healthcare Ltd., Newbury, Berks), were used to induce platelet aggregation. The effect of pre-incubating (5min) PRP (dilution 1:9) with hementin before the addition of inducer was determined. Platelet deaggregation by purified hementin was determined upon its addition to the PRP/inducer mixture when a threshold level of aggregation was reached. Appropriate controls using isotonic phosphate-buffered saline instead of purified hementin were performed with each inducer.

### Platelet aggregation in PRP pre-incubated with hementin

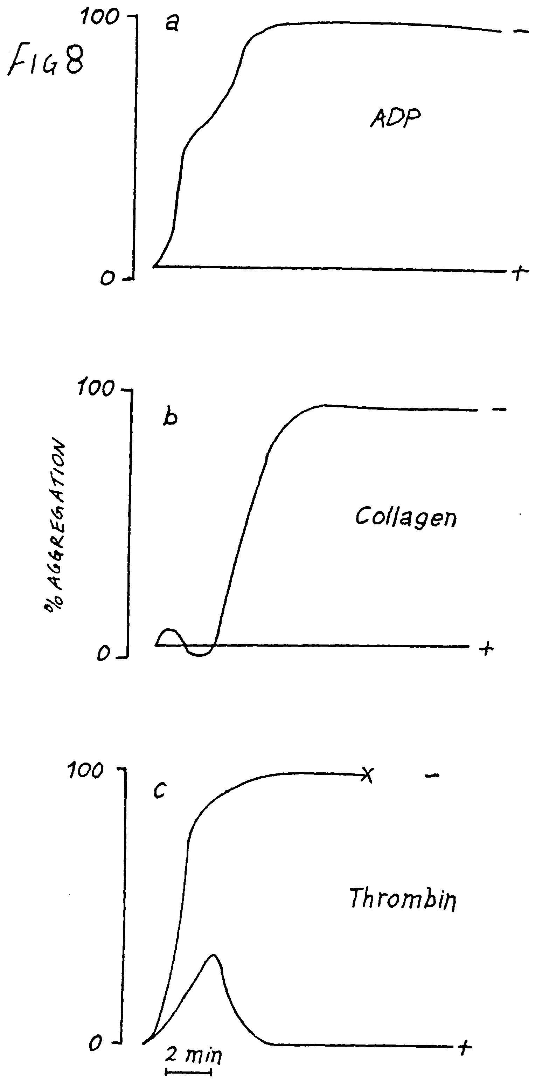

The loss of fibrinogen coagulability by thrombin in PRP pre-incubated with purified hementin, was associated with significant reduction in platelet aggregation responses in both citrated, hirudinised and heparinised blood. Typical responses, which were similar in all five volunteers are shown in Fig.8. Platelet aggregation with 1.0 ug/ml of thrombin as inducer was completely absent in heparinised PRP (Fig. 8c). When citrated plasma was used, the response with 1.0 ug/ml thrombin was reduced to less than 40% of that of the control, with rapid deaggregation and complete loss of subsequent plasma coagulability.

Platelet aggregation responses with collagen (2ug) were almost completely abolished in both citrated and heparinised plasma (Fig. 8b).

Typical aggregation responses with a low concentration of ADP (0.5uM) were similar in citrated and heparinised samples. However, with higher concentrations of ADP (2.5uM), responses in heparinised plasma were different to those in citrate. Whereas platelet aggregation in citrated samples was not affected by hementin at this dose, aggregation responses in heparinised samples were markedly reduced to less than 50% of controls (Fig.8a). The apparent loss of potency of hementin in citrated plasma may be because hementin activity is somewhat inhibited in the presence of citrate.

### Example 14

### Platelet deaggregation by hementin

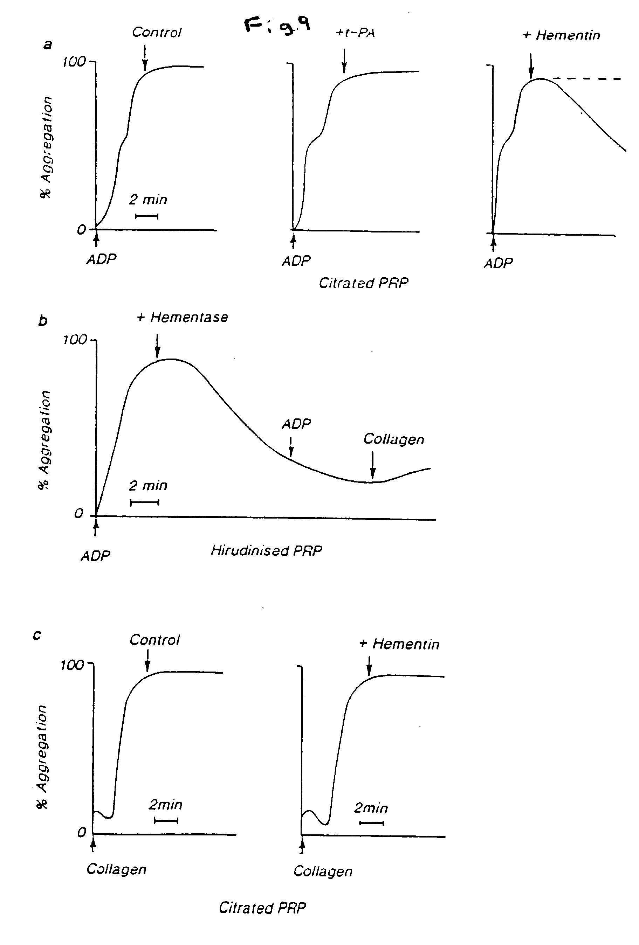

Platelet deaggregation resulting from the addition of hementin to plasma containing aggregated platelets is shown in Fig 9. Platelets in normal human PRP were activated and induced to aggregate through the action of ADP, collagen or thrombin agonists. Hementin was added to these aggregates and the time course and extent of any effect followed in the platelet aggregometer (Fig.9). In citrated or hirudinised plasma ADP at a dose of 1 to 5 uM induced a characteristic (biphasic in the case of citrated plasma) platelet aggregation curve and this was maintained over at least a ten minute period. Addition of hementin (in the concentration range 10 to 100 u/ml) to citrated (Fig 9a) or hirudinised (Fig 9b) ADP-induced platelet aggregates caused rapid and irreversible deaggregation of platelets (Fig 9a,b). In heparinised plasma, a similar observation of deaggregation was made (data not shown). Platelet preparations deaggregated with hementin showed no evidence of change in size distribution compared to unaggregated controls.

Once deaggregation by hementin in hirudinised PRP had taken place, reaggregation by further addition of either ADP (10uM) or collagen (5ug/ml) was not significantly induced (Fig 9b), probably because of fibrinogen depletion within the plasma and/or time necessary for full recovery of platelet function.

In citrated, hirudinised or heparinised PRP, collagen (2ug/ml) induced a characteristic aggregation response. However, hementin (10-100 u/ml), at a dose range effective for ADP induced platelet aggregation (1 to 5 uM), did not cause deaggregation (Fig.9c) in vitro. Higher doses of hementin did deaggregate collagen associated platelet aggregates in vivo.

In citrated plasma, thrombin at a dose of 0.2 ug/ml induced a transient aggregation response in PRP. At a higher agonist concentration of 1 ug/ml, a full aggregation response was obtained with subsequent clotting. Hementin significantly accelerated the spontaneous deaggregation of platelet aggregates following the sub-optimal thrombin dose of 0.2 ug/ml but did not deaggregate platelets aggregated with the higher 1 ug/ml thrombin dose, despite complete prevention of the clot formation in the controls (data not shown). The partial effect of hementin on deaggregation in citrated plasma may be due to some inhibition of hementin by citrate.

### Example 15

### Effect of TPA on platelet deaggregation

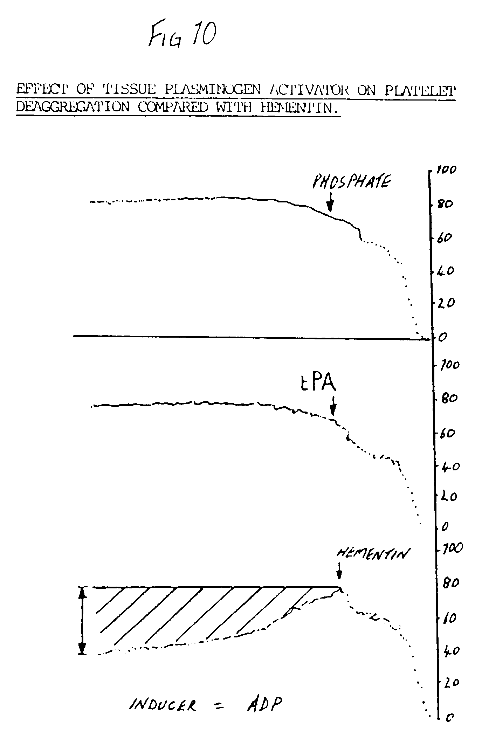

To contrast the effects of hementin on platelet deaggregation with those of another thrombolytic agent, we also found, in a side by side comparison with the same PRP, that TPA at a dose up to 104 IU/ml (sufficient to cause plasma clot lysis within 2 minutes) did not cause platelets to deaggregate in citrated PRP (Figs. 9a, 10). Human platelets were induced to aggregate using ADP (1 to 5 uM) as agonist and TPA (104 IU/ml) or hementin (20 to 70u/ml) were added and the course of deaggregation followed in the platelet aggregometer. This clearly demonstrated that hementin has a mechanism of action different from TPA and that hementin has an added anti-platelet activity which should help in the dissolution of thrombi containing platelets, and other platelet aggregates.

### Example 16

### Enhancement of Hementin Activity in Whole Blood

It is known that hementin is not inhibited by the blood's naturally occurring inhibitors of coagulation proteases (U.S. patent 4390630). We have made the additional significant discovery that the activity of purified hementin is greatly enhanced in whole blood. This finding is surprising and is in marked contrast to the action of TPA which is well known to be inhibited by PA-1 (plasminogen activator inhibitor-l). PA-1 has been implicated as the cause of reduced fibrinolytic capacity of plasma from survivors of myocardial infarctions (Madison, E.E. et al. 1989. Nature 339: 721-724).

By way of illustration, the following experiment was conducted to determine the relative activity of purified hementin in whole blood, plasma (from the same individual) and fibrinogen in buffer. Whole human blood was obtained by venepuncture and anticoagulated with 1:9 v/v 0.1205M sodium citrate. An aliquot of the blood was used to obtain PRP by centrifugation at 3000 rpm for 10 min at room temperature. The reaction buffer used for purified bovine fibrinogen was: 20mM Hepes, 10mM CaCl₂, 0.2M NaCl, 0.1% Brij 35, pH 7.5. Blood and plasma samples were assayed for fibrinogen concentration (normally in the range 2.5 to 4 mg/ml) and the concentration of fibrinogen adjusted comparably in the fibrinogen/buffer experiment.

Citrated human blood, plasma and fibrinogen (adjusted to comparable blood/plasma levels) were incubated with purified hementin (final concentration 10 units/ml) at 37°C. Care was taken to ensure that the hementin preparations were essentially free of an inhibitor of Factor Xa, which is known to contaminate impure preparations. Aliquots were removed at timed intervals, mixed with 2.5 NIH unit bovine thrombin and clotting time recorded.

In parallel experiments the snake fibrinogenolytic enzyme Atroxin was added to aliquots at the same timed intervals to test the integrity of fibrinogen (i.e. Atroxin will not result in a clot if fibrinogen has been previously acted upon by hementin). The results are as follows TT = thrombin clotting time; A = atroxin clotting time):

| | | Clotting time (sec) | | | | |
|---|---|---|---|---|---|---|
| Time | Control | 5 | 10 | 15 | 30 | 60 |
| Whole Blood | | | | | | |
| TT | 15 | >60 | >60 | >60 | >60 | >60 |
| A | 18 | 25 | >60 | >60 | >60 | >60 |

| Plasma | | | | | | |
|---|---|---|---|---|---|---|
| TT | 15 | 15 | 16 | 23 | 30 | >60 |
| A | 18 | 18 | 18 | 24 | 41 | >60 |

| Fibrinogen | | | | | | |
|---|---|---|---|---|---|---|
| TT | 10 | 10 | 10 | 10 | 10 | 10 |
| A | 20 | 20 | 20 | 20 | 20 | 20 |

The same results were obtained with platelet rich plasma (centrifugation of whole blood approximately 200 x g for 10 min).

### Example 17

### Rates of deaggregation and fibrinogenolysis by hementin

Lyophilised hementin was reconstituted with isotonic saline before use for platelet studies or in 20mM Hepes, 10mH CaCl₂, 0.2M NaCl, 0.1% Brij 35, pH 7.5 for in vitro assay. For the measurement of platelet aggregation, PRP was prepared (300 ± 50 x 10⁹ platelets/l) from citrated venous blood of a human volunteer who had received neither medication nor alcohol for at least 2 weeks. Platelet aggregation studies were performed in a four channel aggregometer (Aggregcorder PA3210, Kyoto Daiichi Kagaku Co. Ltd, Japan) at 37 degrees Celsius.

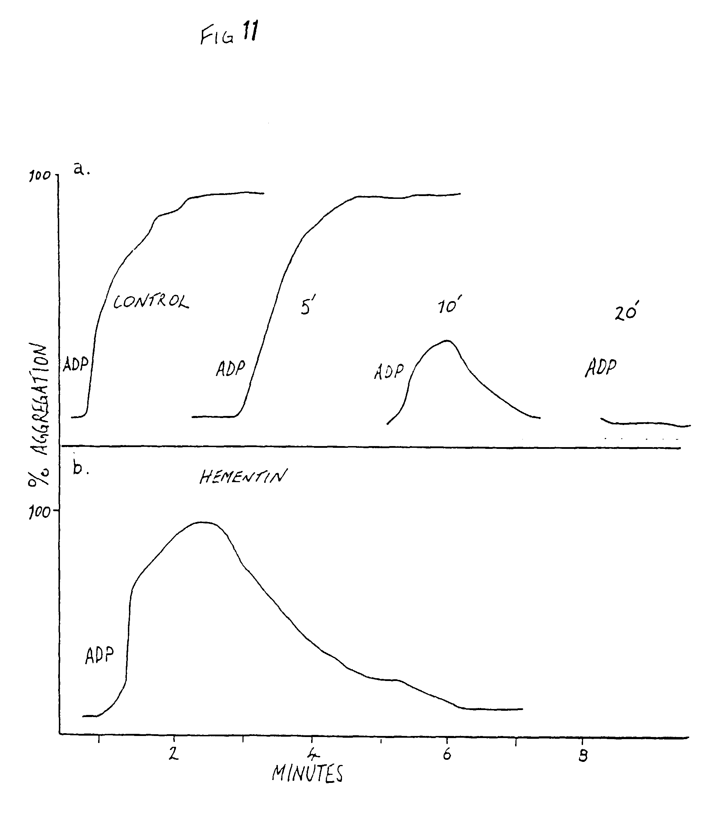

Platelet-rich plasma was pre-incubated at 37 degrees Celsius with purified hementin (10 units/ml) and ADP (2.5um) added at 5,10 and 20 minutes to determine platelet aggregability (results shown in Figure 11a). The same PRP as (a) was aggregated with ADP (2.5um) and hementin (10 units/ml) was added after peak aggregation was attained (results shown in Figure 11b). Controls were also performed to confirm that sufficient ADP had been added to induce and maintain full aggregation over the entire period.

When PRP was incubated at 37 degrees Celsius with hementin (10 units/ml) coagulability with thrombin was lost after 10 minutes. When the ADP-aggregability of the same PRP preparation was also tested at times after addition of hementin (10 units/ml), the ability to aggregate was also lost after 10 minutes. In contrast, the time course of deaggregation induced by purified hementin (10 units/ml) upon pre-formed platelet aggregates (induced with 2.5uM ADP) was much faster with deaggregation essentially complete 3 minutes after addition of hementin. The results are shown in Figure 11. This action of hementin against platelets was complete before any significant effect on coagulation parameters could be measured. Control incubations in the absence of hementin remained fully aggregated.

These results suggest that hementin is able to break down the platelet-fibrinogen-platelet crosslinks faster than it is able to incoagulate plasma. This may be due to the unique cleavage site between the D and E domain of fibrinogen which may allow greater molecular accessibility to hementin than, for example, with plasmin cleavage sites which are predominantly in the carboxyl terminal of fibrinogen and which may be involved in binding to GpIIb-IIIa receptors. Consequently, hementin may be more selective for platelet bound fibrinogen than for free fibrinogen. Since platelet aggregates constitute the bulk of platelet-rich thrombi, which are refractory to current thrombolytic agents based on plasminogen activation, hementin therefore has clinical potential as an agent for deaggregation of platelets within platelet-rich clots causing subsequent thrombolysis.

### Example 18

### Effect of hementin on other fibrinogen-mediated haematological parameters

By virtue of its specific fibrinogenolytic action, purified hementin can be used to determine the contribution of fibrinogen to haematological parameters such as thrombin clotting times and activated partial thromboplastic time (APTT). In a similar manner, hementin can also be used to determine the fibrinogen contribution to the Erythrocyte Sedimentation Rate (ESR) which is a diagnostic marker for certain haematological disorders such as myeloma and rheumatoid arthritis.

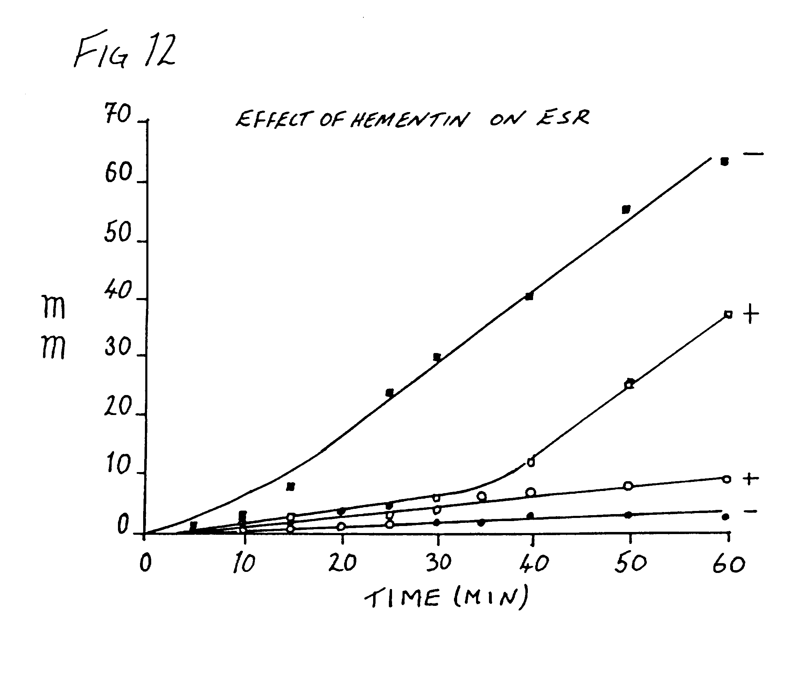

An exemplary experiment, summarised in Figure 12, was carried out as follows. Citrated whole human blood from a volunteer (circles) was incubated for 1h at 25 degrees Celsius (open circles) and without (closed circles) purified hementin (10 units/ml) and the ESR determined. The clotting parameters were determined at PT=95s, APTT=340s; TCT=99s; Atroxin=146s. The results indicate very little effect on ESR despite marked prolongation of clotting parameters for normal blood.

Citrated whole blood from a patient with pulmonary embolism (squares) was incubated for 1h at 25 degrees Celsius with 35 units/ml hementin (open squares) and without purified hementin (closed squares) and the ESR determined. The results indicate that relative to normal values this patient's ESR was markedly accelerated. Incubation with hementin was able to partially reverse this effect leading to slower ESR.

In the example described above, hementin demonstrated marked quantifiable effects on ESR, suggesting that the accelerated ESR observed in patients with pulmonary embolism is in some way owing to erythrocyte interaction with fibrinogen such that the action of hementin leads to reduced ESR. Therefore, hementin can be used as a diagnostic index of the proportion of erythrocyte/fibrinogen in individual patients.

Moreover, hementin reversed the ESR towards normal levels, showing that hementin may be suitable as a therapeutic agent to address the causative factors of abnormal ESR.

Finally, in those cases where increased plasma fibrinogen affects blood viscosity, hementin can be used therapeutically to reduce viscosity. Increased fibrinogen levels in plasma can be raised acutely and chronically in certain diseased states, including infection, third trimester pregnancy, coagulation disorders, liver diseases, post-operative and with age. Reducing elevated fibrinogen levels can lead to reduced risk of clotting.

## Claims

1. Use of a polypetide called hementin for manufacturing a medicament (comprising said polypeptide) for the therapeutic dissolution of pre-formed platelet aggregates including platelet-rich clots in whole blood and *in vivo*, the polypeptide for this is obtainable by homogenizing tissue from the salvary glands of the leech *Heamenteria ghilianii* with solution buffers having salt concentrations of 15 to 25 mM at a pH value between 7 and 8, and purification of the homogenate by ion-exchange chromotography with an eluent having a salt concentration of 80 to 150 mM, and optionally by a gel filtration step.

2. Use according to claim 1 wherein the polypeptide has an isoelectric point of about 6.25 and comprises the following amino acid sequence: 
Thr Thr Leu Thr Glu Pro Glu Pro Phe Leu Thr Tyr Leu Thr Phe Val Xaa Ile Val Xaa
Xaa Glu Met Pro Ile Phe Val Met Ala Thr Ala Asn Ser Gln Ile Thr Ser Thr Phe,
 wherein Xaa equals Arg or Lys (position 17), Asn or Lys (position 20), and Val or Leu (position 21).

3. Use according to claim 1 or 2 wherein the medicament comprises additionally one or more leech derived anti-coagulants.

4. Use according to claim 3, wherein said anticoagulant is selected from anti-thrombin, hirudin, a hirudin analogue, or Factor Xa.

## Patentansprüche

1. Verwendung eines Polypeptids mit der Bezeichnung Hementin zur Herstellung eines Arzneimittels (das dieses Polypeptid enthält) für die therapeutische Auflösung vorgebildeter Blutplättchenaggregate, darunter Gerinsel in Vollblut sowie *in vivo*, die einen hohen Blutplättchengehalt aufweisen, wobei das Polypeptid für diesen Zweck dadurch erhältlich ist, daß man Gewebe aus den Speicheldrüsen des Egels *Heamenteria ghilinaii* mit Lösungspuffern, deren Salzkonzentrationen 15 bis 25 mM betragen, bei einem pH-Wert zwischen 7 und 8 homogenisiert und das Homogenisat durch Ionenaustauschchromotographie mit einem Elutionsmittel, dessen Salzkonzentration 80 bis 150 mM beträgt, reinigt, sowie gegebenenfalls durch einen Gelfiltrationsschritt.

2. Verwendung gemäß Anspruch 1, wobei das Polypeptid über einen isoelektrischen Punkt von ungefähr 6,25 verfügt und die folgende Aminosäuresequenz aufweist: 
Thr Thr Leu Thr Glu Pro Glu Pro Phe Leu Thr Tyr
Leu Thr Phe Val Xaa Ile Val Xaa Xaa Glu Met Pro
Ile Phe Val Met Ala Thr Ala Asn Ser Gln Ile Thr
Ser Thr Phe
 wobei Xaa gleich Arg oder Lys (Position 17), Asn oder Lys (Position 20) sowie Val oder Leu (Position 21) ist.

3. Verwendung gemäß Anspruch 1 oder 2, wobei das Arzneimittel zusätzlich ein oder mehrere Anticoagulantien aus Blutegeln enthält.

4. Verwendung gemäß Anspruch 3, wobei dieses Anticoagulans aus Antithrombin, Hirudin, einem Hirudinanalog oder Faktor Xa ausgewählt ist.

## Revendications

1. Utilisation d'un polypeptide appelé hémentine, pour préparer un médicament (comprenant ledit polypeptide) pour la dissolution thérapeutique d'agrégats plaquettaires préformés, comprenant des caillots riches en plaquettes dans le sang total et in vivo, le polypeptide servant à cette utilisation pouvant être obtenu par homogénéisation d'un tissu provenant des glandes salivaires de la sangsue Heamenteria ghilianii avec des tampons en solution ayant des concentrations de sels de 15 à 25 mM à pH compris entre 7 et 8, et purification de l'homogénéisat par chromatographie par échange d'ions avec un éluant ayant une concentration de sels de 80 à 150 mM, et éventuellement par une étape de filtration sur gel.

2. Utilisation selon la revendication 1, dans laquelle le polypeptide a un point isoélectrique d'environ 6,25 et comprend la séquence d'acides aminés suivante : 
Thr Thr Leu Thr Glu Pro Glu Pro Phe Leu Thr Tyr Leu
Thr Phe Val Xaa Ile Val Xaa Xaa Glu Met Pro Ile Phe Val
Met Ala Thr Ala Asn Ser Gln Ile Thr Ser Thr Phe
 où Xaa représente Arg ou Lys (pposition 17), Asn ou Lys (position 20) et Val ou Leu (position 21).

3. Utilisation selon la revendication 1 ou 2, dans laquelle le médicament comprend en outre un ou deux anti-coagulants dérivés de la sangsue.

4. Utilisation selon la revendication 3, dans laquelle ledit anti-coagulant est choisi parmi l'anti-thrombine, l'hirudine, un analogue de l'hirudine ou le facteur Xa.
